(19) Europäisches Patentamt
European Patent Office
Office européen des brevets

(11) Veröffentlichungsnummer: **0 355 597 B1**

(12) # EUROPÄISCHE PATENTSCHRIFT

(45) Veröffentlichungstag der Patentschrift: **09.06.93**

(51) Int. Cl.[5]: **C07D 317/72**, C07D 413/06, C07D 405/06, C07D 407/06, A01N 43/30

(21) Anmeldenummer: **89114786.0**

(22) Anmeldetag: **10.08.89**

(54) **Substituierte Dioxolanylethylamine, Verfahren zu ihrer Herstellung, ihre Verwendung in Schädlingsbekämpfungsmittteln und neue Zwischenprodukte.**

(30) Priorität: **23.08.88 DE 3828490**

(43) Veröffentlichungstag der Anmeldung: **28.02.90 Patentblatt 90/09**

(45) Bekanntmachung des Hinweises auf die Patenterteilung: **09.06.93 Patentblatt 93/23**

(84) Benannte Vertragsstaaten: **BE CH DE FR GB IT LI NL**

(56) Entgegenhaltungen:
**EP-A- 0 097 822**
**EP-A- 0 131 793**
**EP-A- 0 281 842**
**DE-A- 1 965 321**

**CHEMICAL AND PHARMACEUTICAL BULLETIN, Band 32, Nr. 3, 1984; S.SUGAI et al., Seiten 967-976&NUM;**

(73) Patentinhaber: **BAYER AG**

**W-5090 Leverkusen 1 Bayerwerk(DE)**

(72) Erfinder: **Krämer, Wolfgang, Dr.**
**Rosenkranz 25**
**W-5093 Burscheid 2(DE)**
Erfinder: **Weissmüller, Joachim, Dr.**
**Carl-Langhans-Strasse 53**
**W-4019 Monheim(DE)**
Erfinder: **Berg, Dieter, Dr.**
**Gellertweg 27**
**W-5600 Wuppertal 1(DE)**
Erfinder: **Brandes, Wilhelm, Dr.**
**Eichendorffstrasse 3**
**W-5653 Leichlingen(DE)**
Erfinder: **Dutzmann, Stefan, Dr.**
**Leinenweberweg 33**
**W-4000 Düsseldorf(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft neue substituierte Dioxolanylethylamine, mehrere Verfahren zu ihrer Herstellung, ihre Verwendung in Schädlingsbekämpfungsmitteln und neue Zwischenprodukte.

Es ist bekannt, daß bestimmte Aminomethyldioxolane, wie beispielsweise die Verbindung 2-Isobutyl-2-methyl-4-(1-piperidinylmethyl)-1,3-dioxolan oder die Verbindung 2-Methyl-2-nonyl-4-di-n-butylaminomethyl-1,3-dioxolan oder die Verbindung 2-(2-Cyclohexylmethyl-2-propyl)-2-methyl-4-(1-piperidinylmethyl)-1,3-dioxolan oder die Verbindung 2-(2-Cyclohexylmethyl-2-propyl)-2-methyl-4-(1-perhydroazepinylmethyl)-1,3-dioxolan fungizide Eigenschaften besitzen (vergl. z.B. EP 97 822), weiterhin sind 1,4-Dioxa- bzw. 1,4-Oxathiaspiro(4,5)decane und ihre Verwendung zur Bekämpfung von Schädlingen bekannt (vgl. EP-A 0 281 842).

Die Wirksamkeit dieser vorbekannten Verbindung ist jedoch insbesondere bei niedrigen Aufwandmengen und Konzentrationen nicht in allen Anwendungsbereichen völlig zufriedenstellend.

Es wurden neue substituierte Dioxolanylethylamine der allgemeinen Formel (I),

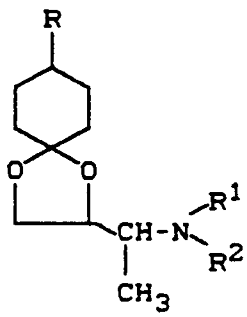

(I)

in welcher

R für Alkyl oder für jeweils gegebenenfalls substituiertes Cycloalkylalkyl, Aralkyl, Cycloalkyl oder Aryl steht,

$R^1$ und $R^2$ unabhängig voneinander jeweils für Wasserstoff, Alkyl, Alkenyl, Alkinyl, Alkoxyalkyl, Dialkoxyalkyl, Hydroxyalkyl, Hydroxyalkoxyalkyl, Alkoxycarbonylalkyl, Dioxolanylalkyl, Dioxanylalkyl, Oxolanylalkyl oder für jeweils gegebenenfalls substituiertes Cycloalkylalkyl, Cycloalkyl, Aralkyl, Aralkenyl oder Aryl stehen oder

$R^1$ und $R^2$ gemeinsam mit dem Stickstoffatom, an welches sie gebunden sind, für einen gegebenenfalls substituierten gesättigten Heterocyclus stehen, der gegebenenfalls weitere Heteroatome enthalten kann,

und deren Säureadditionssalze gefunden.

Die Verbindungen der Formel (I) können als geometrische und/oder optische Isomere oder Isomerengemische unterschiedlicher Zusammensetzung vorliegen. Sowohl die reinen Isomeren als auch die Isomerengemische werden erfindungsgemäß beansprucht. Wenn von Verbindungen der Formel (I) die Rede ist, sind immer alle Formen gemeint.

Weiterhin wurde gefunden, daß man die neuen substituierten Dioxolanylethylamine der allgemeinen Formel (I),

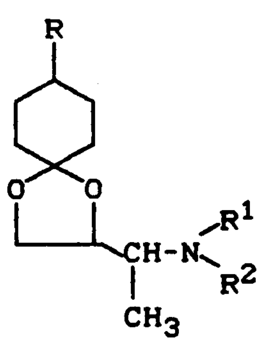

(I)

in welcher

R für Alkyl oder für jeweils gegebenenfalls substituiertes Cycloalkylalkyl, Aralkyl, Cycloalkyl oder Aryl steht,

$R^1$ und $R^2$ unabhängig voneinander jeweils für Wasserstoff, Alkyl, Alkenyl, Alkinyl, Alkoxyalkyl, Dialkoxyalkyl, Hydroxyalkyl, Hydroxyalkoxyalkyl, Alkoxycarbonylalkyl, Dioxolanylalkyl, Dioxanylalkyl, Oxolanylalkyl oder für jeweils gegebenenfalls substituiertes Cycloalkylalkyl, Cycloalkyl, Aralkyl, Aralkenyl oder Aryl stehen oder

$R^1$ und $R^2$ gemeinsam mit dem Stickstoffatom, an welches sie gebunden sind, für einen gegebenenfalls substituierten gesättigten Heterocyclus stehen, der gegebenenfalls weitere Heteroatome enthalten kann,

und deren Säureadditionssalze erhält, wenn man

(a) substituierte Dioxolane der Formel (II),

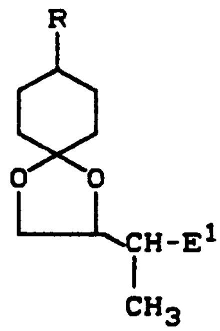

(II)

in welcher

R die oben angegebene Bedeutung hat und

$E^1$ für eine elektronenanziehende Abgangsgruppe steht,

mit Aminen der Formel (III),

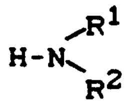

(III)

in welcher

$R^1$ und $R^2$ die oben angegebene Bedeutung haben,

gegebenenfalls in Gegenwart eines Verdünnungsmittels und gegebenenfalls in Gegenwart eines Säurebindemittels umsetzt, oder wenn man

(b) die nach Verfahren (a) erhältlichen erfindungsgemäßen substituierten Dioxolanylethylamine der Formel (Ia),

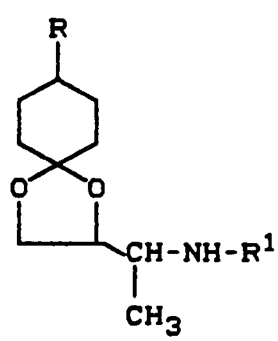

(Ia)

in welcher

R und $R^1$ die oben angegebene Bedeutung haben,
mit Alkylierungsmitteln der Formel (IV),

$R^{2-1}$-$E^2$ (IV)

in welcher

$R^{2-1}$ für Alkyl, Alkenyl, Alkinyl, Alkoxyalkyl, Dialkoxyalkyl, Hydroxyalkyl, Hydroxyalkoxyalkyl, Alkoxycarbonylalkyl, Dioxolanylalkyl, Dioxanylalkyl, Oxolanylalkyl oder für jeweils gegebenenfalls substituiertes Cycloalkylalkyl, Cycloalkyl, Aralkyl oder Aralkenyl steht und

$E^2$ für eine elektronenanziehende Abgangsgruppe steht,

gegebenenfalls in Gegenwart eines Verdünnungsmittels und gegebenenfalls in Gegenwart eines Säurebindemittels umsetzt
und gegebenenfalls anschließend eine Säure addiert oder eine physikalische Trennmethode anschließt.

Schließlich wurde gefunden, daß die neuen substituierten Dioxolanylethylamine der allgemeinen Formel (I) eine Wirkung gegen Schädlinge, insbesondere gegen pilzliche Schädlinge besitzen.

Überraschenderweise zeigen die erfindungsgemäßen substituierten Dioxolanylethylamine der allgemeinen Formel (I) eine bessere fungizide Wirksamkeit als die aus dem Stand der Technik bekannten Aminomethyldioxolane, wie beispielsweise die Verbindung 2-Isobutyl-2-methyl-4-(1-piperidinylmethyl)-1,3-dioxolan oder die Verbindung 2-Methyl-2-nonyl-4-di-n-butylaminomethyl-1,3-dioxolan oder die Verbindung 2-(2-Cyclohexylmethyl-2-propyl)-2-methyl-4-(1-piperidinylmethyl)-1,3-dioxolan oder die Verbindung 2-(2-Cyclohexylmethyl-2-propyl)-2-methyl-4-(1-perhydroazepinylmethyl)-1,3-dioxolan, welche chemisch und wirkungsmäßig naheliegende Verbindungen sind.

Die erfindungsgemäßen substituierten Dioxolanylethylamine sind durch die Formel (I) allgemein definiert. Bevorzugt sind Verbindungen der Formel (I), bei welchen

R für geradkettiges oder verzweigtes Alkyl mit 1 bis 12 Kohlenstoffatomen, für jeweils gegebenenfalls einfach bis mehrfach, gleich oder verschieden durch Halogen und/oder geradkettiges oder verzweigtes Alkyl mit 1 bis 8 Kohlenstoffatomen substituiertes Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach bis mehrfach, gleich oder verschieden durch Halogen und/oder geradkettiges oder verzweigtes Alkyl mit 1 bis 8 Kohlenstoffatomen substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht und

$R^1$ und $R^2$ unabhängig voneinander jeweils für Wasserstoff, für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, für Alkoxycarbonylalkyl mit 1 bis 6 Kohlenstoffatomen je Alkoxy- bzw. Alkylteil, für jeweils geradkettiges oder verzweigtes Dioxolanylalkyl, Dioxanylalkyl oder Oxolanylalkyl mit jeweils 1 bis 4 Kohlenstoffatomen im Alkylteil oder für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil stehen, wobei als Substituenten jeweils infrage kommen:
Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen;
außerdem für jeweils gegebenenfalls einfach bis mehrfach, gleich oder verschieden im Arylteil substituiertes Arylalkyl, Arylalkenyl oder Aryl mit jeweils 6 bis 10 Kohlenstoffstoffatomen im Arylteil und gegebenenfalls bis zu 6 Kohlenstoffatomen im geradkettigen oder verzweigten Alkyl- bzw. Alkenylteil stehen, wobei als Arylsubstituenten jeweils infrage kommen:
Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder

$R^1$ und $R^2$ gemeinsam mit dem Stickstoffatom, an welches sie gebunden sind, für einen gegebenenfalls einfach bis mehrfach, gleich oder verschieden substituierten, gesättigten 5- bis 7-gliedrigen Heterocyclus stehen, der gegebenenfalls ein weiteres Heteroatom, insbesondere Stickstoff, Sauerstoff oder Schwefel enthalten kann, wobei als Substituenten infrage kommen:

jeweils geradkettiges oder verzweigtes Alkyl oder Hydroxyalkyl mit jeweils 1 bis 4 Kohlenstoffatomen.

Besonders bevorzugt sind Verbindungen der Formel (I), bei welchen

R für geradkettiges oder verzweigtes Alkyl mit 1 bis 8 Kohlenstoffatomen, für gegebenenfalls ein- bis dreifach, gleich oder verschieden durch Chlor, Methyl, Ethyl, n- oder i-Propyl, n-, i-, s- oder t-Butyl substituiertes Cyclohexyl, für im Cyclohexylteil gegebenenfalls ein- bis dreifach, gleich oder verschieden durch Chlor, Methyl, Ethyl, n- oder i-Propyl, n-, i-, s- oder t-Butyl substituiertes Cyclohexylalkyl mit 1 bis 4 Kohlenstoffatomen im geradkettigen oder verzweigten Alkylteil, für gegebenenfalls ein- bis dreifach, gleich oder verschieden durch Fluor, Chlor, Brom, Methyl, Ethyl, n- oder i-Propyl, n-, i-, s- oder t-Butyl substituiertes Phenyl oder für im Phenylteil gegebenenfalls ein- bis dreifach, gleich oder verschieden durch Fluor, Chlor, Brom, Methyl, Ethyl, n- oder i-Propyl, n-, i-, s- oder t-Butyl substituiertes Phenylalkyl mit 1 bis 4 Kohlenstoffatomen im geradkettigen oder verzweigten Alkylteil steht und

$R^1$ und $R^2$ unabhängig voneinander jeweils für Wasserstoff, Methyl, Ethyl, n- oder i-Propyl, n-, i-, s- oder t-Butyl, n- oder i-Pentyl, n- oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Propoxycarbonylpropyl, Dioxolanylmethyl, Dioxolanylethyl, Dioxanylmethyl, Dioxanylethyl, Oxolanylmethyl, Oxolanylethyl, für jeweils gegebenenfalls ein-bis fünffach, gleich oder verschieden durch Fluor, Chlor, Brom, Methyl, Ethyl, n- oder i-Propyl, n-, i-, s- oder t-Butyl, Trifluormethyl oder Trifluormethoxy substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Phenyl, Benzyl oder Phenylethyl stehen, wobei als Substituenten jeweils infrage kommen:

Fluor, Chlor, Brom, Cyano, Nitro, Methyl, Ethyl, n-oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

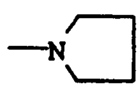 ; 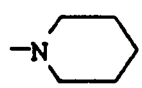 ; 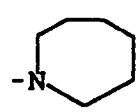 ; 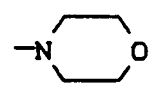

oder 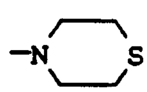

stehen, wobei als Substituenten jeweils infrage kommen: Methyl, Ethyl oder Hydroxymethyl.

Ganz besonders bevorzugt sind Verbindungen der Formel (I), bei welchen

R für Cyclohexyl, Phenyl oder für einen der Reste $-CH_2-R^3$,

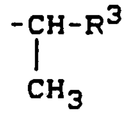 oder 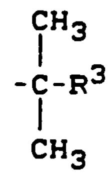

steht, wobei

$R^3$ jeweils für Wasserstoff, Methyl, Ethyl, n-oder i-Propyl, n-, i-, s- oder t-Butyl, Neopentyl, Cyclohexyl oder Phenyl steht,

$R^1$ und $R^2$ unabhängig voneinander jeweils für Wasserstoff, Methyl, Ethyl, n- oder i-Propyl, n-, i-, s- oder t-Butyl, n- oder i-Pentyl, n- oder i-Hexyl, Allyl, n- oder i-Butenyl, n- oder i-Pentenyl, Propargyl, n- oder i-Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Propoxycarbonylpropyl, Dioxolanylmethyl, Dioxolanylethyl, Dioxanylmethyl, Oxolanylmethyl, Oxolanylethyl, Cyclopropylmethyl, Dichlorcyclopropylmethyl, Dimethylcyclopropylmethyl, Dichlordimethylcyclopropylmethyl, Cyclopentyl, Cyclohexyl oder Cyclohexylmethyl stehen oder

$R^1$ und $R^2$ gemeinsam mit dem Stickstoffatom, an welches sie gebunden sind, für einen gegebenenfalls ein-bis dreifach, gleich oder verschieden substituierten Heterocyclus der Formel

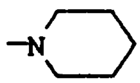 ; 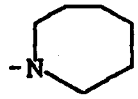 ; 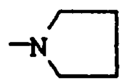 oder 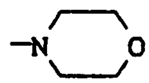

stehen,
wobei als Substituenten jeweils infrage kommen:
Methyl, Ethyl oder Hydroxymethyl.

Bevorzugte erfindungsgemäße Verbindungen sind auch Additionsprodukte aus Säuren und denjenigen substituierten Dioxolanylethylaminen der Formel (I), in denen die Substituenten R, $R^1$ und $R^2$ die Bedeutungen haben, die bereits für diese Substituenten genannt wurden.

Zu den Säuren die addiert werden können und die zu pflanzenverträglichen Additionsprodukten führen, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie p-Toluolsulfonsäure und 1,5-Naphthalindisulfonsäure sowie Saccharin oder Thiosaccharin.

Verwendet man beispielsweise 8-s-Butyl-2-(1-chlorethyl)-1,4-dioxanspiro[4,5]decan und Piperidin als Ausgangsstoffe, so läßt sich der Reaktionsablauf des erfindungsgemäßen Verfahrens (a) durch das folgende Formelschema darstellen:

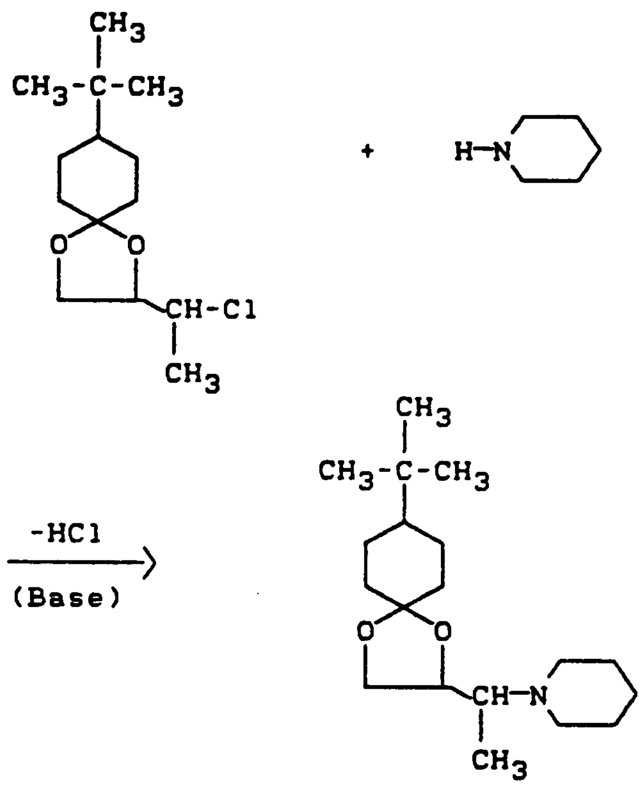


Verwendet man beispielsweise 8-t-Butyl-2-(1-methylamino)-ethyl-1,4-dioxaspiro[4,5]decan und Allylbromid als Ausgangsstoffe, so läßt sich der Reaktionsablauf des erfindungsgemäßen Verfahrens (b) durch das folgende Formelschema darstellen:

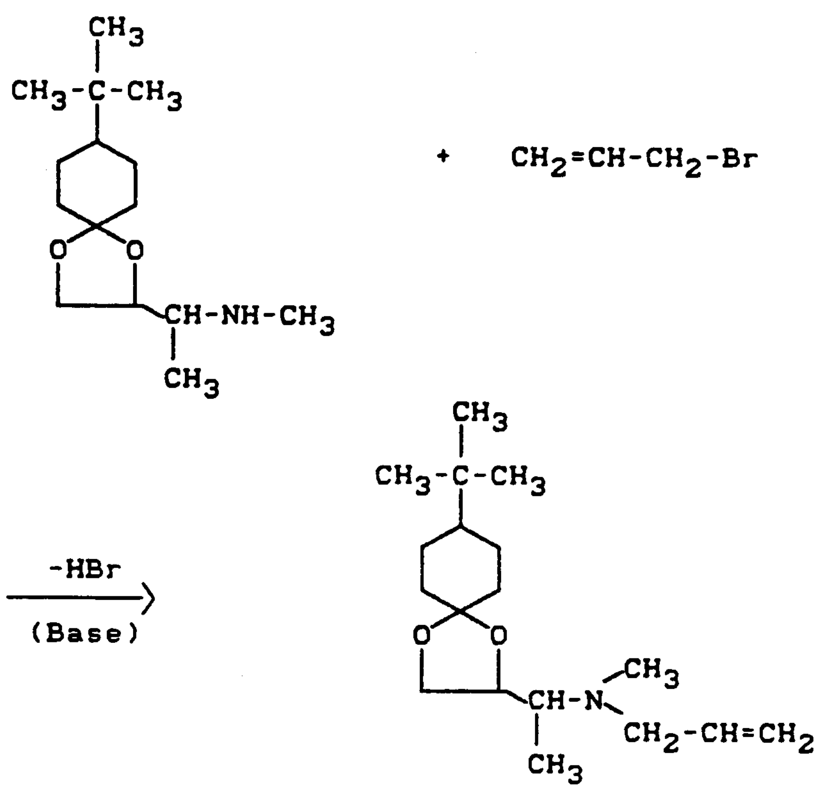

Die zur Durchführung des erfindungsgemäßen Verfahrens (a) als Ausgangsstoffe benötigten substituierten Dioxolane sind durch die Formel (II) allgemein definiert. In dieser Formel (II) steht R vorzugsweise für diejenigen Reste, die bereits im Zusammenhang mit der Beschreibung der erfindungsgemäßen Stoffe der Formel (I) für diese Substituenten genannt wurden.

$E^1$ steht vorzugsweise für Halogen, insbesondere für Iod, Chlor oder Brom oder für gegebenenfalls durch Halogen, wie Fluor, Chlor, Brom oder Iod substituiertes Alkylsulfonyloxy oder für gegebenenfalls durch Alkyl mit 1 bis 4 Kohlenstoffatomen substituiertes Arylsulfonyloxy, wie beispielsweise Methansulfonyloxy, Trifluormethansulfonyloxy oder p-Toluolsulfonyloxy.

Die substituierten Dioxolane der Formel (II) sind noch nicht bekannt und ebenfalls Gegenstand der Erfindung. Man erhält sie in Analogie zu bekannten Verfahren (vergl. z.B. Liebigs Ann. Chem. 1984, 1298-1301; Z. Naturforsch. B, Anorg. Chem., Org. Chem. 4013, 393-397 [1985] oder J. org. Chem. 51, 1894-1897 [1986] sowie die Herstellungsbeispiele), beispielsweise wenn man Cyclohexanon-Derivate der Formel (V),

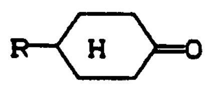

(V)

in welcher

R die oben angegebene Bedeutung hat,

entweder

(a) mit Alkoholen der Formel (VIa),

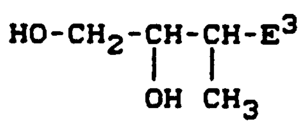

(VIa)

in welcher

$E^3$ für Halogen oder Hydroxy steht,

gegebenenfalls in Gegenwart eines Verdünnungsmittels, wie beispielsweise Toluol, und gegebenenfalls in Gegenwart eines sauren Katalysators, wie beispielsweise p-Toluolsulfonsäure, bei Temperaturen zwischen 40 °C und 150 °C cyclisiert

oder

(b) mit Oxiranylverbindungen der Formel (VIb),

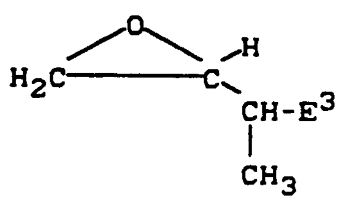

(VIb)

in welcher

$E^3$ für Halogen oder Hydroxy steht,

gegebenenfalls in Gegenwart eines Verdünnungsmittels, wie beispielsweise Tetrachlorkohlenstoff, und gegebenenfalls in Gegenwart eines Katalysators, wie beispielsweise Zinntetrachlorid, bei Temperaturen zwischen -20 °C und +40 °C umsetzt und gegebenenfalls in den Fällen, wo $E^3$ in Formel (VIa) oder (VIb) für eine Hydroxygruppe steht, in einer 2. Stufe die so erhältlichen Hydroxymethyldioxolane der Formel (VII),

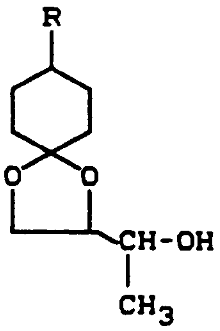

(VII)

in welcher
R die oben angegebene Bedeutung hat,
mit gegebenenfalls substituierten Alkyl- oder Arylsulfonylhalogeniden der Formel (VIII),

$Z\text{-}SO_2\text{-}Hal$ (VIII)

in welcher
Hal für Halogen, insbesondere für Chlor steht und
Z für jeweils gegebenenfalls durch Halogen, wie Fluor, Chlor, Brom oder Iod substituiertes Alkyl oder für gegebenenfalls durch Alkyl mit 1 bis 4 Kohlenstoffatomen substituiertes Aryl, wie insbesondere Methyl, Trifluormethyl oder 4-Methylphenyl steht,

gegebenenfalls in Gegenwart eines Verdünnungsmittels, wie beispielsweise Diethylether, und gegebenenfalls in Gegenwart eines Säurebindemittels, wie beispielsweise Pyridin oder Triethylamin, bei Temperaturen zwischen -20 °C und +100 °C umsetzt.

Die dabei erhältlichen geometrischen Isomeren bzw. Diastereomeren lassen sich entweder als Gemische im erfindungsgemäßen Verfahren (a) weiter umsetzen oder mit üblichen Trennmethoden (Chromatographie, Kristallisation) auftrennen.

Die Cyclohexanon-Derivate der Formel (V) sind bekannt oder lassen sich in Analogie zu bekannten Verfahren herstellen (vergl. z.B. Tetrahedron Letters 28, 2347-2350 [1987]; Tetrahedron Lett. 27, 2875-2878 [1986]; Tetrahedron Lett. 1979, 3209-3212; J. Am. chem. Soc. 109, 6887-6889 [1987]; J. Am. chem. Soc. 95, 3646-3651 [1973]; J. Am. chem. Soc. 94, 7599-7600 [1972]; Bull. chem. Soc. Jap. 60, 1721-1726 [1987]; Chem. Lett. 1986, 1593-1596; Synth. Commun. 15, 759-764 [1985]; Synth. Commun. 12, 267-277 [1982]; J. chem. Soc.; Chem. Commun. 1984, 762-763; J. org. Chem. 38, 1775-1776 [1973]; US 4 251 398; US 3 960 961; EP 2136; DE 26 36 684; DE 25 09 183; FR 22 31 650 sowie die Herstellungsbeispiele).

Die Alkohole der Formel (VIa) sind ebenfalls bekannt (vergl. z.B. EP 200 267; DE- 29 37 840; US 4 035 178; Tetrahedron 27, 3197-3205 [1971]; Tetrahedron 35, 2583-2589 [1979]; Carbohydrate Res. 31, 17-26 [1973]).

Die Oxiranylverbindungen der Formel (VIb) sind ebenfalls bekannt (vergl. z.B. EP 3664; J.Am. chem. Soc. 96, 5254-5255 [1974]; Tetrahedron Lett. 1977, 4397-4400; Tetrahedron Lett. 1979, 4733-4736; Tetrahedron Lett. 21. 4843-4846 [1980]).

Die Sulfonylhalogenide der Formel (VIII) sind allgemein bekannte Verbindungen der organischen Chemie.

Die zur Durchführung des erfindungsgemäßen Verfahrens (a) weiterhin als Ausgangsstoffe benötigten Amine sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) für diese Substituenten genannt wurden.

Die Amine der Formel (III) sind allgemein bekannte Verbindungen der organischen Chemie.

Die zur Durchführung des erfindungsgemäßen Verfahrens (b) als Ausgangsstoffe benötigten substituierten Dioxolanylethylamine sind durch die Formel (Ia) allgemein definiert. In dieser Formel (Ia) stehen R und $R^1$ vorzugsweise für diejenigen Reste, die bereits im Zusammenhang mit der Beschreibung der erfindungsgemäßen Stoffe der Formel (I) für diese Substituenten genannt wurden.

Die substituierten Dioxolanylethylamine der Formel (Ia) sind erfindungsgemäße Verbindungen und erhältlich mit Hilfe des erfindungsgemäßen Verfahrens (a).

Die zur Durchführung des erfindungsgemäßen Verfahrens (b) weiterhin als Ausgangsstoffe benötigten Alkylierungsmittel sind durch die Formel (IV) allgemein definiert. In dieser Formel (IV) steht $R^{2-1}$ vorzugs-

weise für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit 1 bis 6 Kohlenstoffatomen oder Hydroxyalkoxyalkyl mit 2 bis 6 Kohlenstoffatomen in den einzelnen Alkylteilen, für Alkoxycarbonylalkyl mit 1 bis 6 Kohlenstoffatomen je Alkoxy- bzw. Alkylteil, für jeweils geradkettiges oder verzweigtes Dioxolanylalkyl, Dioxanylalkyl oder Oxolanylalkyl mit jeweils 1 bis 4 Kohlenstoffatomen im Alkylteil, für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil, wobei als Substituenten jeweils infrage kommen: Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes Arylalkyl oder Arylalkenyl mit jeweils 6 bis 10 Kohlenstoffatomen im Arylteil und bis zu 6 Kohlenstoffatomen im geradkettigen oder verzweigten Alkyl- bzw. Alkenylteil, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen.

$R^{2-1}$ steht besonders bevorzugt für Methyl, Ethyl, n-oder i-Propyl, n-, i-, s- oder t-Butyl, n- oder i-Pentyl, n- oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Dioxolanylmethyl, Dioxolanylethyl, Dioxanylmethyl, Dioxanylethyl, Oxolanylmethyl, Oxolanylethyl, für gegebenenfalls jeweils ein- bis fünffach, gleich oder verschieden durch Fluor, Chlor, Brom, Methyl, Ethyl, n- oder i-Propyl, n-, i-, s- und t-Butyl, Trifluormethyl oder Trifluormethoxy substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Benzyl oder Phenylethyl, wobei als Substituenten jeweils infrage kommen:

Fluor, Chlor, Brom, Cyano, Nitro, Methyl, Ethyl, n-oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl.

$R^{2-1}$ steht ganz besonders bevorzugt für Methyl, Ethyl, n- oder i-Propyl, n-, i-, s- oder t-Butyl, n- oder i-Pentyl, n- oder i-Hexyl, Allyl, n- oder i-Butenyl, n- oder i-Pentenyl, Propargyl, n- oder i-Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Propoxycarbonylpropyl, Dioxolanylmethyl, Dioxolanylethyl, Dioxanylmethyl, Cyclopropylmethyl, Oxolanylmethyl, Oxolanylethyl, Dichlorcyclopropylmethyl, Dimethylcyclopropylmethyl, Dichlordimethylcyclopropylmethyl, Cyclopentyl, Cyclohexyl oder Cyclohexylmethyl.

$E^2$ steht vorzugsweise für Halogen, insbesondere für Chlor, Brom oder Iod oder für jeweils gegebenenfalls durch Halogen, wie Fluor, Chlor, Brom oder Iod substituiertes Alkylsulfonyloxy oder Alkoxysulfonyloxy mit jeweils 1 bis 4 Kohlenstoffatomen oder für gegebenenfalls durch Alkyl mit 1 bis 4 Kohlenstoffatomen substituiertes Arylsulfonyloxy, wie beispielsweise Methansulfonyloxy, Methoxysulfonyloxy oder p-Toluolsulfonyloxy.

Die Alkylierungsmittel der Formel (IV) sind ebenfalls allgemein bekannte Verbindungen der organischen Chemie oder erhältlich in Analogie zu allgemein bekannten Verfahren.

Als verdünnungsmittel zur Durchführung der erfindungsgemäßen Verfahren (a) und (b) kommen inerte organische Lösungsmittel oder wäßrige Systeme infrage. Hierzu gehören insbesondere aliphatische oder aromatische, gegebenenfalls halogenierte Kohlenwasserstoffe, wie biespielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid; Ester, wie Essigsäureethylester; Sulfoxide, wie Dimethylsulfoxid oder Alkohole, wie Methanol, Ethanol oder Propanol.

Die erfindungsgemäßen Verfahren (a) und (b) können gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyldimethyl-ammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid oder Trimethylbenzylammoniumchlorid. Es ist auch möglich die erfindungsgemäßen Verfahren (a) und (b) ohne Zusatz eines Lösungsmittels durchzuführen.

Als Säurebindemittel zur Durchführung der erfindungsgemäßen Verfahren (a) und (b) kommen alle üblicherweise verwendbaren anorganischen und organischen Basen infrage. Vorzugsweise verwendet man Alkalimetallhydroxide, -alkoholate, -carbonate oder -hydrogencarbonate, wie beispielsweise Natriumhydroxid, Natriummethylat, Natriumethylat, Natriumcarbonat oder Natriumhydrogencarbonat oder auch tertiäre Amine, wie beispielweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Es ist auch möglich, die als Reaktionsteilnehmer verwendeten Amine der Formeln (III) bzw. (Ia) in entsprechendem Überschuß gleichzeitig als Säurebindemittel einzusetzen.

Die Reaktionstemperaturen können bei der Durchführung der erfindungsgemäßen Verfahren (a) und (b) in einem größeren Bereich variiert werden. Im allgemeinen arbeitet man bei Temperaturen zwischen +20 °C und +200 °C, vorzugsweise bei Temperaturen zwischen 80 °C und +180 °C.

Die erfindungsgemäßen Verfahren (a) und (b) werden im allgemeinen bei Normaldruck durchgeführt. Es ist jedoch auch möglich unter erhöhtem Druck im Bereich zwischen 1 und 20 bar zu arbeiten. Die Arbeitsweise unter erhöhtem Druck empfiehlt sich insbesondere, wenn ein oder mehrere Reaktionsteilnehmer bei Normaldruck und der erforderlichen Reaktionstemperatur gasförmig vorliegen.

Zur Durchführung des erfindungsgemäßen Verfahrens (a) setzt man pro Mol an substituiertem Heterocyclus der Formel (II) im allgemeinen 1,0 bis 10,0 Mol, vorzugsweise 1,0 bis 5,0 Mol an Amin der Formel (III) und gegebenenfalls 1,0 bis 10,0 Mol, vorzugsweise 1,0 bis 5,0 Mol an Säurebindemittel, und gegebenenfalls 0,1 bis 1,0 Mol an Phasentransferkatalysator ein.

Zur Durchführung des erfindungsgemäßen Verfahrens (b) setzt man pro Mol an Dioxolanylethylamin der Formel (Ia) im allgemeinen 1,0 bis 5,0 Mol, vorzugsweise 1,0, bis 2,0 Mol an Alkylierungsmittel der Formel (IV) und 1,0 bis 5,0 Mol, vorzugsweise 1,0 bis 2,0 Mol an Säurebindemittel, und gegebenenfalls 0,1 bis 1,0 Mol an Phasentransferkatalysator ein.

Die Reaktionsdurchführung, Aufarbeitung und Isolierung der Reaktionsprodukte der Formel (I) erfolgt in beiden Fällen nach üblichen Methoden.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus

(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger der Braunspelzigkeit des Weizens (Leptosphaeria nodorum) oder gegen den Erreger der Blattfleckenkrankheit der Gerste (Pyrenophora teres) oder gegen den Erreger der Blattfleckenkrankheit des Weizens (Cochliobolus sativus) sowie gegen Mehltau und Rostarten oder zur Bekämpfung von Krankheiten im Obst- und Gemüseanbau, wie beispielsweise gegen den Erreger des Apfelschorfes (Venturia inaequalis), oder gegen den Erreger des Gurkenmehltaus (Sphaerotheca fuliginea) oder zur Bekämpfung von Krankheiten im Reisanbau, wie beispielsweise gegen den Erreger der Reisfleckenkrankheit (Pyricularia oryzae) einsetzen.

Darüber hinaus zeigen die erfindungsgemäßen Wirkstoffe eine gute in-vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1:

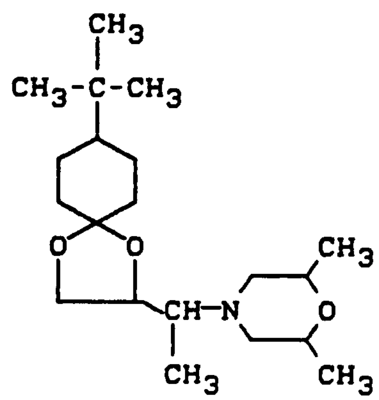

(Verfahren a)

Eine Mischung aus 14 g (0,054 Mol) 8-t-Butyl-2-(1-chlorethyl)-1,4-dioxaspiro[4,5]decan, 11 g (0,096 Mol) cis-2,6-Dimethylmorpholin, 12 g (0,087 Mol) Kaliumcarbonat und 1 g Kaliumiodid in 100 ml Ethanol wird für 20 Stunden bei einem Druck von 14 bar auf 180 °C erhitzt. Nach dem Abkühlen wird die Reaktionsmischung eingeengt, der Rückstand in Dichlormethan aufgenommen, mehrfach mit Wasser gewaschen, über Natriumsulfat getrocknet, im Vakuum eingeengt und der Rückstand über Kieselgel mit Petrolether/Essigester (10 : 1) chromatographiert.

Man erhält 2,2 g eines Isomeren (I) mit den $^{1}H$-NMR Daten: δ(ppm): 4,0-4,15 (m, 2H), 3,75-3,85 (m,1H) und 1,1 (d,3H) und 1,2 g eines Isomeren (II) vom Brechungsindex $n_D^{20}$ 1,4745 des 8-t-Butyl-2-[1-(cis-2,6-dimethyl-4-morpholinyl)-ethyl]-1,4-dioxaspiro[4,5]decan (18,5 % der Theorie an Gesamtausbeute).

Herstellung der Ausgangsverbindung

Beispiel II-1:

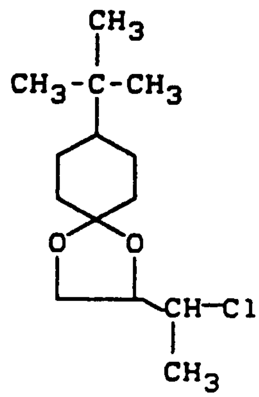

Zu einer Lösung von 38,2 g (0,36 Mol) 1,2-Epoxi-3-chlorbutan und 44 g (0,286 Mol) 4-t-Butylcyclohexanon in 100 ml Tetrachlorkohlenstoff tropft man unter Rühren eine Lösung von 15 g (0,057 Mol) Zinntetrachlorid in 40 ml Tetrachlorkohlenstoff, so daß die Temperatur der Reaktionsmischung 32 °C nicht übersteigt. Nach beendeter Zugabe rührt man 4 Stunden bei Raumtemperatur, verdünnt dann mit 100 ml Toluol, wäscht nacheinander mit 200 ml 10 prozentiger Natronlauge und Wasser, trocknet über Kaliumcarbonat und Natriumsulfat und entfernt das Lösungsmittel im Vakuum.

Man erhält 71 g (74 % der Theorie) an 8-t-Butyl-2-(1-chlorethyl)-1,4-dioxaspiro[4,5]decan als Isomerengemisch vom Brechungsindex $n_D^{20}$ 1,4740.

In entsprechender Weise und gemäß den allgemeinen Angaben zur Herstellung erhält man die folgenden substituierten Dioxolanylethylamine der allgemeinen Formel (I):

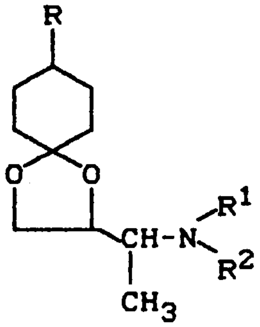

(I)

| Bsp. Nr. | R | 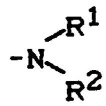 | physikalische Eigenschaften |
|---|---|---|---|
| 2 | $(CH_3)_3C-$ | 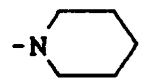 | $n_D^{20}$ 1,4836 (Isomeres I) |
| 3 | $(CH_3)_3C-$ | 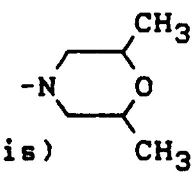 (cis) | $n_D^{20}$ 1,4765 (Isomeren-gemisch) |
| 4 | $(CH_3)_3C-$ | 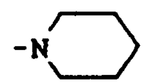 | $n_D^{20}$ 1,4791 (Isomeres II) |
| 5 | $(CH_3)_3C-$ | 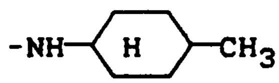 | $n_D^{20}$ 1,4840 (Isomeres I) |
| 6 | $(CH_3)_3C-$ | 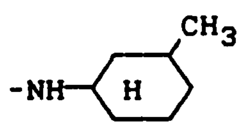 | $n_D^{20}$ 1,4834 (Isomeres II) |
| 7 | $(CH_3)_3C-$ | 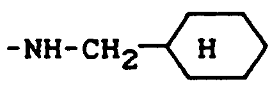 | $n_D^{20}$ 1,4732 (Isomeres I) |

| Bsp. Nr. | R | 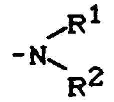 | physikalische Eigenschaften |
|---|---|---|---|
| 8 | $(CH_3)_3C-$ | 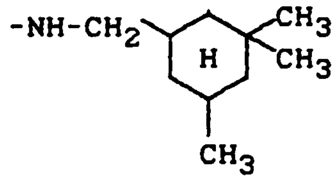 | $n_D^{20}$ 1,4796 (Isomeres I) |
| 9 | $(CH_3)_3C-$ | 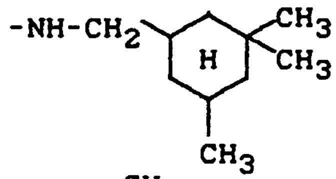 | $n_D^{20}$ 1,4765 (Isomeres II) |
| 10 | $(CH_3)_3C-$ | 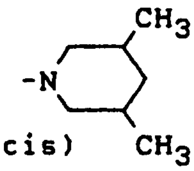 (cis) | $^1$H-NMR*): 3,95-4,1(m,2H) 3,75-3,85(m,1H) 1,02 (d,3H) |
| 11 | $(CH_3)_3C-$ | $-NH-CH_2-CH(C_2H_5)_2$ | $n_D^{20}$ 1,4716 (Isomeres I) |
| 12 | $(CH_3)_3C-$ | $-NH-CH_2-CH(C_2H_5)_2$ | $n_D^{20}$ 1,4683 (Isomerengemisch) |
| 13 | $(CH_3)_3C-$ | 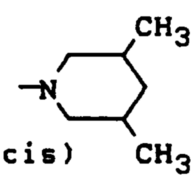 (cis) | $n_D^{20}$ 1,4743 (Isomeres II) |
| 14 | $(CH_3)_3C-$ | 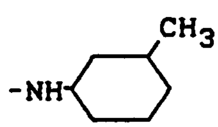 | $n_D^{20}$ 1,4810 (Isomerengem.) |
| 15 | $(CH_3)_3C-$ | 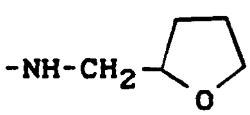 | $n_D^{20}$ 1,4908 (Isomerengem.) |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

## Anwendungsbeispiele

In den folgenden Anwendungsbeispielen wurden die nachstehend aufgeführten Verbindungen als Vergleichssubstanzen eingesetzt:

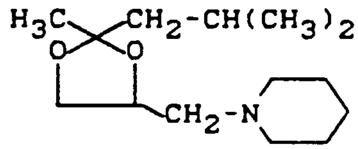

(A)

2-Isobutyl-2-methyl-4-(1-piperidinylmethyl)-1,3-dioxolan

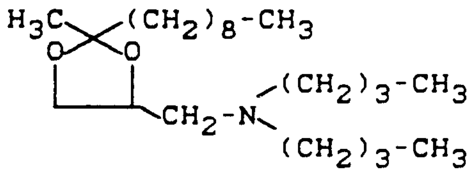

(B)

2-Methyl-2-nonyl-4-di-n-butylaminomethyl-1,3-dioxolan

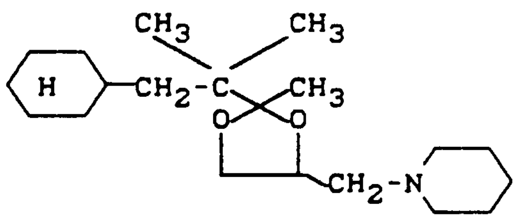

(C)

2-(2-Cyclohexylmethyl-2-propyl)-2-methyl-4-(1-piperidinylmethyl)-1,3-dioxolan

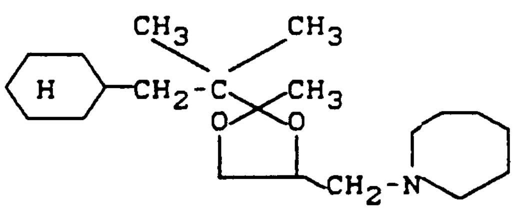

(D)

2-(2-Cyclohexylmethyl-2-propyl)-2-methyl-4-(1-perhydroazepinylmethyl)-1,3-dioxolan (alle bekannt aus EP 97 822).

Beispiel A

Leptosphaeria nodorum-Test (Weizen)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80% aufgestellt.

10 Tage nach der Inokulation erfolgt die Auswertung.

Eine deutlich Überlegenheit in der Wirksamkeit gegenüber dem Stand der Technik zeigen bei diesem Test z.B. die Verbindungen gemäß den Herstellungsbeispielen: 3, 4, 5, 6, 7 und 11.

Beispiel B

Sphaerotheca-Test (Gurke) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24 ° C und bei einer relativen Luftfeuchtigkeit von ca. 75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindung gemäß Herstellungsbeispiel 8.

**Patentansprüche**

**1.** Substituierte Dioxolanylethylamine der allgemeinen Formel (I),

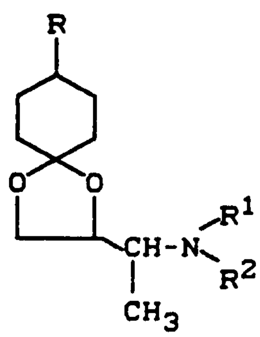

(I)

in welcher

R für Alkyl oder für jeweils gegebenenfalls substituiertes Cycloalkylalkyl, Aralkyl, Cycloalkyl oder Aryl steht,

$R^1$ und $R^2$ unabhängig voneinander jeweils für Wasserstoff, Alkyl, Alkenyl, Alkinyl, Alkoxyalkyl, Dialkoxyalkyl, Hydroxyalkyl, Hydroxyalkoxyalkyl, Alkoxycarbonylalkyl, Dioxolanylalkyl, Dioxanylalkyl, Oxolanylalkyl oder für jeweils gegebenenfalls substituiertes Cycloalkylalkyl, Cycloalkyl, Aralkyl, Aralkenyl oder Aryl stehen oder

$R^1$ und $R^2$ gemeinsam mit dem Stickstoffatom, an welches sie gebunden sind, für einen gegebenenfalls substituierten gesättigten Heterocyclus stehen, der gegebenenfalls weitere Heteroatome enthalten kann,

und deren Säureadditionssalze.

**2.** Substituierte Dioxolanylethylamine gemäß Anspruch 1, wobei in der Formel (I)

R für geradkettiges oder verzweigtes Alkyl mit 1 bis 12 Kohlenstoffatomen, für jeweils gegebenenfalls einfach bis mehrfach, gleich oder verschieden durch Halogen und/oder geradkettiges oder verzweigtes Alkyl mit 1 bis 8 Kohlenstoffatomen substituiertes Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach bis mehrfach, gleich oder verschieden

durch Halogen und geradkettiges oder verzweigtes Alkyl mit 1 bis 8 Kohlenstoffatomen substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht und

$R^1$ und $R^2$ unabhängig voneinander jeweils für Wasserstoff, für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, für Alkoxycarbonylalkyl mit 1 bis 6 Kohlenstoffatomen je Alkoxy-bzw. Alkylteil, für jeweils geradkettiges oder verzweigtes Dioxolanylalkyl, Dioxanylalkyl oder Oxolanylalkyl mit jeweils 1 bis 4 Kohlenstoffatomen im Alkylteil oder für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil stehen, wobei als Substituenten jeweils infrage kommen: Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen;

außerdem für jeweils gegebenenfalls einfach bis mehrfach, gleich oder verschieden im Arylteil substituiertes Arylalkyl, Arylalkenyl oder Aryl mit jeweils 6 bis 10 Kohlenstoffstoffatomen im Arylteil und gegebenenfalls bis zu 6 Kohlenstoffatomen im geradkettigen oder verzweigten Alkyl-bzw. Alkenylteil stehen, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder

$R^1$ und $R^2$ gemeinsam mit dem Stickstoffatom, an welches wie gebunden sind, für einen gegebenenfalls einfach bis mehrfach, gleich oder verschieden substituierten, gesättigten 5- bis 7-gliedrigen Heterocyclus stehen, der gegebenenfalls ein weiteres Heteroatom enthalten kann, wobei als Substituenten infrage kommen: jeweils geradkettiges oder verzweigtes Alkyl oder Hydroxyalkyl mit jeweils 1 bis 4 Kohlenstoffatomen.

**3.** Substituierte Dioxolanylethylamine gemäß Anspruch 1, wobei

R für geradkettiges oder verzweigtes Alkyl mit 1 bis 8 Kohlenstoffatomen, für gegebenenfalls ein- bis dreifach, gleich oder verschieden durch Chlor, Methyl, Ethyl, n- oder i-Propyl, n-, i-, s- oder t-Butyl substituiertes Cyclohexyl, für im Cyclohexylteil gegebenenfalls ein- bis dreifach, gleich oder verschieden durch Chlor, Methyl, Ethyl, n-oder i-Propyl, n-, i-, s- oder t-Butyl substituiertes Cyclohexylalkyl mit 1 bis 4 Kohlenstoffatomen im geradkettigen oder verzweigten Alkylteil, für gegebenenfalls ein-bis dreifach, gleich oder verschieden durch Fluor, Chlor, Brom, Methyl, Ethyl, n- oder i-Propyl, n-, i-, s-oder t-Butyl substituiertes Phenyl oder für im Phenylteil gegebenenfalls ein- bis dreifach, gleich oder verschieden durch Fluor, Chlor, Brom, Methyl, Ethyl, n-oder i-Propyl, n-, i-, s- oder t-Butyl substituiertes Phenylalkyl mit 1 bis 4 Kohlenstoffatomen im geradkettigen oder verzweigten Alkylteil steht und

$R^1$ und $R^2$ unabhängig voneinander jeweils für Wasserstoff, Methyl, Ethyl, n- oder i-Propyl, n-, i-, s- oder t-Butyl, n- oder i-Pentyl, n- oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Propoxycarbonylpropyl, Dioxolanylmethyl, Dioxolanylethyl, Dioxanylmethyl, Dioxanylethyl, Oxolanylmethyl, Oxolanylethyl, für jeweils gegebenenfalls ein- bis fünffach, gleich oder verschieden durch Fluor, Chlor, Brom, Methyl, Ethyl, n- oder i-Propyl, n-, i-, s- oder t-Butyl, Trifluormethyl oder Trifluormethoxy substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl

oder für jeweils gegebenenfalls ein- bis dreifach, gleich oder verschieden substituiertes Phenyl, Benzyl oder Phenylethyl stehen, wobei als Substituenten jeweils infrage kommen: Fluor, Chlor, Brom, Cyano, Nitro, Methyl, Ethyl, n-oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

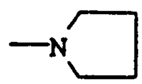 ; 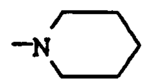 ; 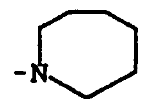 ; 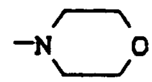

oder 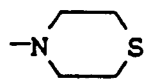

stehen, wobei als Substituenten jeweils infrage kommen: Methyl, Ethyl oder Hydroxymethyl.

**4.** Substituierte Dioxolanylethylamine gemäß Anspruch 1, wobei in der Formel (I)

R für Cyclohexyl, Phenyl oder für einen der Reste
$-CH_2-R^3$,

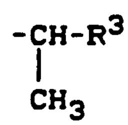 oder 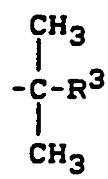

steht, wobei

$R^3$ jeweils für Wasserstoff, Methyl, Ethyl, n-oder i-Propyl, n-, i-, s- oder t-Butyl, Neopentyl, Cyclohexyl oder Phenyl steht,

$R^1$ und $R^2$ unabhängig voneinander jeweils für Wasserstoff, Methyl, Ethyl, n- oder i-Propyl, n-, i-, s-oder t-Butyl, n- oder i-Pentyl, n- oder i-Hexyl, Allyl, n- oder i-Butenyl, n- oder i-Pentenyl, Propargyl, n- oder i-Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Propoxycarbonylpropyl, Dioxolanylmethyl, Dioxolanylethyl, Dioxanylmethyl, Oxolanylmethyl, Oxolanylethyl, Cyclopropylmethyl, Dichlorcyclopropylmethyl, Dimethylcyclopropylmethyl, Dichlordimethylcyclopropylmethyl, Cyclopentyl, Cyclohexyl oder Cyclohexylmethyl stehen oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

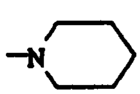 ; 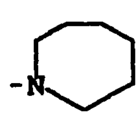 ; 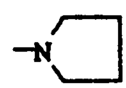 oder 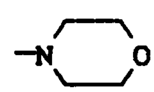

stehen,
wobei als Substituenten jeweils infrage kommen: Methyl, Ethyl oder Hydroxymethyl.

**5.** Verfahren zur Herstellung von substituierten Dioxolanylethylamin der allgemeinen Formel (I),

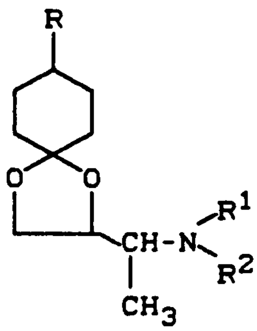

(I)

in welcher

R für Alkyl oder für jeweils gegebenenfalls substituiertes Cycloalkylalkyl, Aralkyl, Cycloalkyl oder Aryl steht,

$R^1$ und $R^2$ unabhängig voneinander jeweils für Wasserstoff, Alkyl, Alkenyl, Alkinyl, Alkoxyalkyl, Dialkoxyalkyl, Hydroxyalkyl, Hydroxyalkoxyalkyl, Alkoxycarbonylalkyl, Dioxolanylalkyl, Dioxanylalkyl, Oxolanylalkyl oder für jeweils gegebenenfalls substituiertes Cycloalkylalkyl, Cycloalkyl, Aralkyl, Aralkenyl oder Aryl stehen oder

$R^1$ und $R^2$ gemeinsam mit dem Stickstoffatom, an welches sie gebunden sind, für einen gegebenenfalls substituierten gesättigten Heterocyclus stehen, der gegebenenfalls weitere Heteroatome enthalten kann,

und deren Säureadditionssalze, dadurch gekennzeichnet, daß man

(a) substituierte Dioxolane der Formel (II),

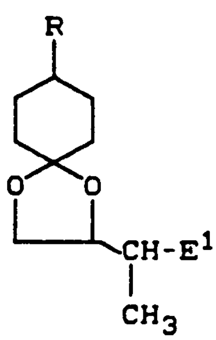

(II)

in welcher

R die oben angegebene Bedeutung hat und

$E^1$ für eine elektronenanziehende Abgangsgruppe steht,

mit Aminen der Formel (III),

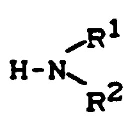

(III)

in welcher

$R^1$ und $R^2$ die oben angegebene Bedeutung haben,

gegebenenfalls in Gegenwart eines Verdünnungsmittels und gegebenenfalls in Gegenwart eines Säurebindemittels umsetzt, oder

(b) die nach Verfahren (a) erhältlichen erfindungsgemäßen substituierten Dioxolanylethylamine der Formel (Ia),

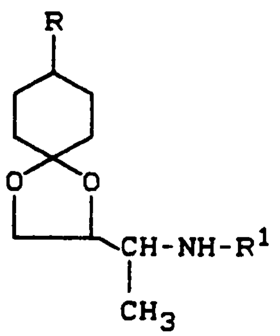

(Ia)

in welcher

R und $R^1$ die oben angegebene Bedeutung haben,

mit Alkylierungsmitteln der Formel (IV),

$R^{2-1}$-$E^2$ (IV)

in welcher

$R^{2-1}$ für Alkyl, Alkenyl, Alkinyl, Alkoxyalkyl, Dialkoxyalkyl, Hydroxyalkyl, Hydroxyalkoxyalkyl, Alkoxycarbonylalkyl, Dioxolanylalkyl, Dioxanylalkyl, Oxolanylalkyl oder für jeweils gegebenenfalls substituiertes Cycloalkylalkyl, Cycloalkyl, Aralkyl oder Aralkenyl steht und

$E^2$ für eine elektronenanziehende Abgangsgruppe steht,

gegebenenfalls in Gegenwart eines Verdünnungsmittels und gegebenenfalls in Gegenwart eines Säurebindemittels umsetzt und gegebenenfalls eine Säure addiert oder eine physikalische Trennmethode anschließt.

**6.** Schädlingsbekämpfungsmittel, gekennzeichnet durch einen Gehalt an mindestens einem substituierten Dioxolanylethylamin der Formel (I) nach den Ansprüchen 1 oder 5.

**7.** Verwendung von substituierten Dioxolanylethylaminen der Formel (I) nach den Ansprüchen 1 oder 5 zur Bekämpfung von Schädlingen.

**8.** Verfahren zur Bekämpfung von Schädlingen, dadurch gekennzeichnet, daß man mindestens ein substituiertes Dioxolanylethylamin der Formel (I) nach den Ansprüchen 1 oder 5 auf Schädlinge und/oder ihren Lebensraum einwirken läßt.

**9.** Verfahren zur Herstellung von Schädlingsbekämpfungsmitteln, dadurch gekennzeichnet, daß man mindestens ein substituiertes Dioxolanylethylamin der Formel (I) nach den Ansprüchen 1 oder 5 mit Streckmitteln und/oder oberflächenaktiven Mitteln vermischt.

**10.** Substituierte Dioxolane der Formel (II),

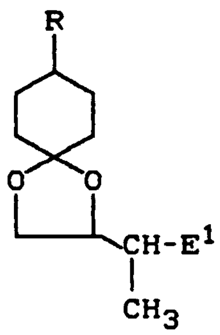

(II)

in welcher

R für Alkyl oder für jeweils gegebenenfalls substituiertes Cycloalkylalkyl, Aralkyl, Cycloalkyl oder Aryl steht und

$E^1$ für eine elektronenanziehende Abgangsgruppe steht.

**11.** Verfahren zur Herstellung von substituierten Dioxolanen der Formel (II),

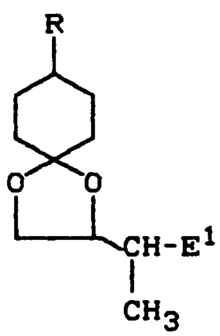

(II)

in welcher

R für Alkyl oder für jeweils gegebenenfalls substituiertes Cycloalkylalkyl, Aralkyl, Cycloalkyl oder Aryl steht und

$E^1$ für eine elektronenanziehende Abgangsgruppe steht,

dadurch gekennzeichnet, daß man Cyclohexanon-Derivate der Formel (V),

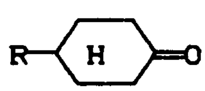

(V)

in welcher

R die oben angegebene Bedeutung hat,

entweder

(a) mit Alkoholen der Formel (VIa),

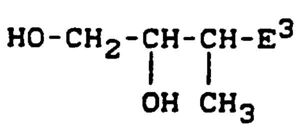

(VIa)

in welcher

$E^3$ für Halogen oder Hydroxy steht,
gegebenenfalls in Gegenwart eines Verdünnungsmittels und gegebenenfalls in Gegenwart eines sauren Katalysators bei Temperaturen zwischen 40 °C und 150 °C cyclisiert
oder
(b) mit Oxiranylverbindungen der Formel (VIb),

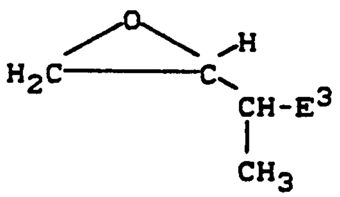

(VIb)

in welcher
$E^3$ für Halogen oder Hydroxy steht,
gegebenenfalls in Gegenwart eines Verdünnungsmittels und gegebenenfalls in Gegenwart eines Katalysators bei Temperaturen zwischen -20 °C und +40 °C umsetzt und gegebenenfalls in den Fällen, wo $E^3$ in Formel (VIa) oder (VIb) für eine Hydroxygruppe steht, in einer 2. Stufe die so erhältlichen Hydroxymethyldioxolane der Formel (VII),

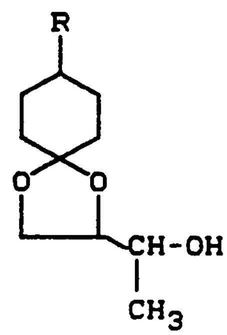

(VII)

in welcher
R die oben angegebene Bedeutung hat,
mit gegebenenfalls substituierten Alkyl- oder Arylsulfonylhalogeniden der Formel (VIII),

$Z\text{-}SO_2\text{-}Hal$ (VIII)

in welcher
Hal für Halogen steht und
Z für jeweils gegebenenfalls durch Halogen substituiertes Alkyl oder für gegebenenfalls durch Alkyl mit 1 bis 4 Kohlenstoffatomen substituiertes Aryl steht,
gegebenenfalls in Gegenwart eines Verdünnungsmittels und gegebenenfalls in Gegenwart eines Säurebindemittels bei Temperaturen zwischen -20 °C und +100 °C umsetzt.

## Claims

1. Substituted dioxolanylethylamines of the general formula (I)

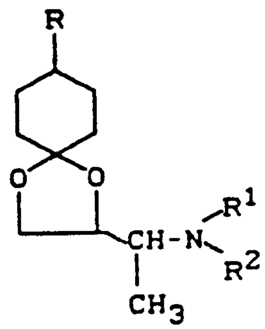

(I)

in which

R represents alkyl, or represents in each case optionally substituted cycloalkylalkyl, aralkyl, cycloalkyl or aryl,

$R^1$ and $R^2$ independently of one another each represent hydrogen, alkyl, alkenyl, alkinyl, alkoxyalkyl, dialkoxyalkyl, hydroxyalkyl, hydroxyalkoxyalkyl, alkoxycarbonylalkyl, dioxolanylalkyl, dioxanylalkyl or oxolanylalkyl, or represent in each case optionally substituted cycloalkylalkyl, cycloalkyl, aralkyl, aralkenyl or aryl, or

$R^1$ and $R^2$, together with the nitrogen atom to which they are bonded, represent an optionally substituted saturated heterocyclic radical, which can optionally contain further hetero atoms, and acid addition salts thereof.

2. Substituted dioxolanylethylamines according to Claim 1, wherein, in formula (I),

R represents straight-chain or branched alkyl having 1 to 12 carbon atoms, or represents cycloalkylalkyl or cycloalkyl having in each case 3 to 7 carbon atoms in the cycloalkyl part and if appropriate 1 to 6 carbon atoms in the straight-chain or branched alkyl part and in each case optionally substituted by one or more identical or different substituents from the group comprising halogen and/or straight-chain or branched alkyl having 1 to 8 carbon atoms, or represents aralkyl or aryl having in each case 6 to 10 carbon atoms in the aryl part and if appropriate 1 to 6 carbon atoms in the straight-chain or branched alkyl part and in each case optionally substituted by one or more identical or different substitutents from the group comprising halogen and straight-chain or branched alkyl having 1 to 8 carbon atoms, and

$R^1$ and $R^2$ independently of one another each represent hydrogen, or represent in each case straight-chain or branched alkyl having 1 to 12 carbon atoms, alkenyl having 3 to 8 carbon atoms, alkinyl having 3 to 8 carbon atoms, hydroxyalkyl having 2 to 6 carbon atoms, alkoxyalkyl or dialkoxyalkyl having in each case 1 to 6 carbon atoms or hydroxyalkoxyalkyl having 2 to 6 carbon atoms in the individual alkyl parts, or represent alkoxycarbonylalkyl having 1 to 6 carbon atoms per alkoxy and alkyl part, or represent in each case straight-chain or branched dioxolanylalkyl, dioxanylalkyl or oxolanylalkyl having in each case 1 to 4 carbon atoms in the alkyl part, or represent cycloalkyl or cycloalkylalkyl having in each case 3 to 7 carbon atoms in the cycloalkyl part and if appropriate 1 to 4 carbon atoms in the straight-chain or branched alkyl part and in each case optionally substituted in the cycloalkyl part by one or more identical or different substituents, possible substituents in each case being: halogen, and in each case straight-chain or branched alkyl, alkoxy, halogenoalkyl or halogenoalkoxy having in each case 1 to 4 carbon atoms and if appropriate 1 to 9 identical or different halogen atoms;

or furthermore represent arylalkyl, arylalkenyl or aryl having in each case 6 to 10 carbon atoms in the aryl part and if appropriate up to 6 carbon atoms in the straight-chain or branched alkyl or alkenyl part and in each case optionally substituted in the aryl part by one or more identical or different substituents, possible substituents on

the aryl in each case being: halogen, cyano, nitro and in each case straight-chain or branched alkyl, alkoxy, alkylthio, halogenoalkyl, halogenoalkoxy, halogenoalkylthio, alkoxycarbonyl or alkoximinoalkyl having in each case 1 to 4 carbon atoms in the individual alkyl parts and if appropriate 1 to 9 identical or different halogen atoms, or

$R^1$ and $R^2$, together with the nitrogen atom to which they are bonded, represent a saturated 5- to 7-membered heterocyclic radical which is optionally substituted by one or more identical or different substituents and can optionally contain a further hetero atom, possible substituents being: in each case straight-chain or branched alkyl or hydroxyalkyl having in each case 1 to 4 carbon atoms.

**3.** Substituted dioxolanylethylamines according to Claim 1, wherein

R represents straight-chain or branched alkyl having 1 to 8 carbon atoms, or represents cyclohexyl which is optionally substituted by one to three identical or different substituents from the group comprising chlorine, methyl, ethyl, n- or i-propyl and n-, i-, s- or t-butyl, or represents cyclohexylalkyl having 1 to 4 carbon atoms in the straight-chain or branched alkyl part and optionally substituted in the cyclohexyl part by one to three identical or different substituents from the group comprising chlorine, methyl, ethyl, n- or i-propyl and n-, i-, s- or t-butyl, or represents phenyl which is optionally substituted by one to three identical or different substituents from the group comprising fluorine, chlorine, bromine, methyl, ethyl, n- or i-propyl, n-, i-, s- or t-butyl, or represents phenylalkyl having 1 to 4 carbon atoms in the straight-chain or branched alkyl part and optionally substituted in the phenyl part by one to three identical or different substituents from the group comprising fluorine, chlorine, bromine, methyl, ethyl, n- or i-propyl and n-, i-, s- or t-butyl, and

$R^1$ and $R^2$ independently of one another each represent hydrogen, methyl, ethyl, n- or i-propyl, n-, i-, s- or t-butyl, n- or i-pentyl, n- or i-hexyl, n- or i-heptyl, n- or i-octyl, allyl, n- or i-butenyl, n- or i-pentenyl, propargyl, n- or i-butinyl, hydroxyethyl, hydroxypropyl, methoxyethyl, ethoxyethyl, propoxyethyl, butoxyethyl, methoxypropyl, ethoxypropyl, propoxypropyl, butoxypropyl, hydroxyethoxyethyl, dimethoxyethyl, dimethoxypropyl, diethoxyethyl, methoxycarbonylmethyl, methoxycarbonylethyl, methoxycarbonylpropyl, ethoxycarbonylmethyl, ethoxycarbonylethyl, ethoxycarbonylpropyl, propoxycarbonylmethyl, propoxycarbonylethyl, propoxycarbonylpropyl, dioxolanylmethyl, dioxolanylethyl, dioxanylmethyl, dioxanylethyl, oxolanylmethyl or oxolanylethyl, or represent cyclopropyl, cyclopropylmethyl, cyclopropylethyl, cyclopropylpropyl, cyclopentyl, cyclopentylmethyl, cyclohexyl or cyclohexylmethyl, in each case optionally substituted by one to five identical or different substituents from the group comprising fluorine, chlorine, bromine, methyl, ethyl, n- or i-propyl, n-, i-, s- or t-butyl, trifluoromethyl or trifluoromethoxy, or represent phenyl, benzyl or phenylethyl, in each case optionally substituted by one to three identical or different substituents, possible substituents in each case being: fluorine, chlorine, bromine, cyano, nitro, methyl, ethyl, n- or i-propyl, n-, i-, s- or t-butyl, methoxy, ethoxy, methylthio, trifluoromethyl, trifluoromethoxy, trifluoromethylthio, methoxycarbonyl, ethoxycarbonyl or methoximinomethyl, or

$R^1$ and $R^2$, together with the nitrogen atom to which they are bonded, represent a heterocyclic radical of the formula

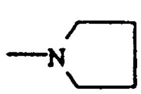 ; 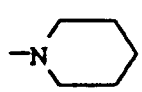 ; 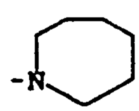 ; 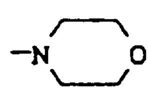

or 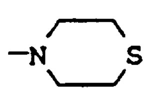

which is optionally substituted by one to three identical or different substituents, possible substituents in each case being: methyl, ethyl or hydroxymethyl.

**4.** Substituted dioxolanylethylamines according to Claim 1, wherein, in formula (I),

R represents cyclohexyl or phenyl, or represents one of the radicals $-CH_2-R^3$,

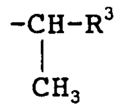 or 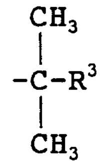 ,

wherein

$R^3$ in each case represents hydrogen, methyl, ethyl, n- or i-propyl, n-, i-, s- or t-butyl, neopentyl, cyclohexyl or phenyl,

$R^1$ and $R^2$ independently of one another each represent hydrogen, methyl, ethyl, n- or i-propyl, n-, i-, s- or t-butyl, n- or i-pentyl, n- or i-hexyl, allyl, n- or i-butenyl, n- or i-pentenyl, propargyl, n- or i-butinyl, hydroxyethyl, hydroxypropyl, methoxyethyl, methoxypropyl, ethoxyethyl, ethoxypropyl, hydroxyethoxyethyl, dimethoxyethyl, diethoxyethyl, methoxycarbonylmethyl, methoxycarbonylethyl, methoxycarbonylpropyl, ethoxycarbonylmethyl, ethoxycarbonylethyl, ethoxycarbonylpropyl, propoxycarbonylmethyl, propoxycarbonylethyl, propoxycarbonylpropyl, dioxolanylmethyl, dioxolanylethyl, dioxanylmethyl, oxolanylmethyl, oxolanylethyl, cyclopropylmethyl, dichlorocyclopropylmethyl, dimethylcyclopropylmethyl, dichlorodimethylcyclopropylmethyl, cyclopentyl, cyclohexyl or cyclohexylmethyl, or

$R^1$ and $R^2$, together with the nitrogen atom to which they are bonded, represent a heterocyclic radical of

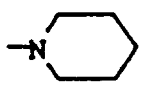 ; 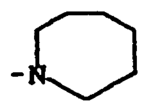 ; 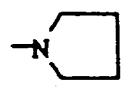 oder 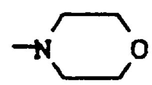

which is optionally substituted by one to three identical or different substituents, possible substituents in each case being: methyl, ethyl or hydroxymethyl.

**5.** Process for the preparation of substituted dioxolanylethylamines of the general formula (I)

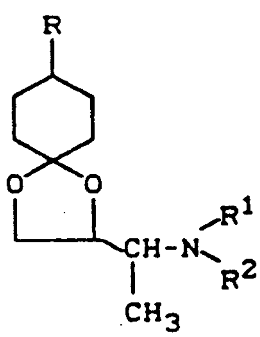

(I)

in which

R represents alkyl, or represents in each case optionally substituted cycloalkylalkyl, aralkyl, cycloalkyl or aryl,

$R^1$ and $R^2$ independently of one another each represent hydrogen, alkyl, alkenyl, alkinyl, alkoxyalkyl, dialkoxyalkyl, hydroxyalkyl, hydroxyalkoxyalkyl, alkoxycarbonylalkyl, dioxolanylalkyl, dioxanylalkyl or oxolanylalkyl, or represent in each case optionally substituted cycloalkylalkyl, cycloalkyl, aralkyl, aralkenyl or aryl, or

$R^1$ and $R^2$, together with the nitrogen atom to which they are bonded, represent an optionally substituted saturated heterocyclic radical, which can optionally contain further hetero atoms,

and acid addition salts thereof, characterised in that

(a) substituted dioxolanes of the formula (II)

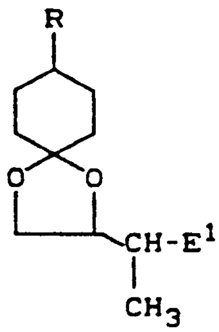

(II)

in which

R has the abovementioned meaning and

$E^1$ represents an electron-withdrawing leaving group,

are reacted with amines of the formula (III)

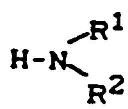

(III)

in which

$R^1$ and $R^2$ have the abovementioned meaning,

if appropriate in the presence of a diluent and if appropriate in the presence of an acid-binding agent, or by a process in which

(b) the substituted dioxolanylethylamines according to the invention, which are obtainable by process (a), of the formula (Ia)

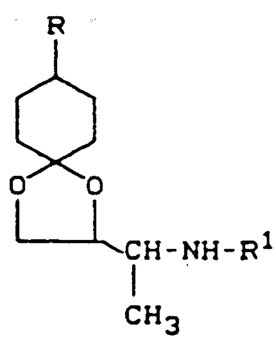

(Ia)

in which

R and $R^1$ have the abovementioned meaning,

are reacted with alkylating agents of the formula (IV)

$R^{2-1}$-$E^2$ (IV)

in which

$R^{2-1}$ represents alkyl, alkenyl, alkinyl, alkoxyalkyl, dialkoxyalkyl, hydroxyalkyl, hydroxyalkoxyalkyl, alkoxycarbonylalkyl, dioxolanylalkyl, dioxanylalkyl or oxolanylalkyl, or represents in each case optionally substituted cycloalkylalkyl, cycloalkyl, aralkyl or aralkenyl and

$E^2$ represents an electron-withdrawing leaving group,

if appropriate in the presence of a diluent and if appropriate in the presence of an acid-binding agent, and if appropriate an acid is then added on or the process is followed by a physical resolution method.

**6.** Agents for combating pests, characterized in that they contain at least one substituted dioxolanylethylamine of the formula (I) according to Claims 1 or 5.

**7.** Use of substituted dioxolanylmethylamines of the formula (I) according to Claims 1 or 5 for combating pests.

**8.** Method of combating pests, characterized in that at least one substituted dioxolanylethylamine of the formula (I) according to Claims 1 or 5 is allowed to act on pests and/or their environment.

**9.** Process for the preparation of agents for combating pests, characterized in that in that at least one substituted dioxolanylethylamine of the formula (I) according to Claims 1 or 5 is mixed with extenders and/or surface-active agents.

**10.** Substituted dioxolanes of the formula (II)

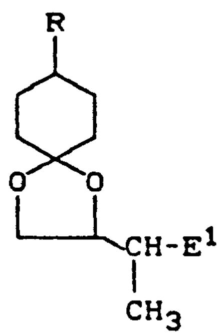

II

in which

R represents alkyl, or represents in each case optionally substituted cycloalkylalkyl, aralkyl, cycloalkyl or aryl and

$E^1$ represents an electron-withdrawing leaving group.

**11.** Process for the preparation of substituted dioxolanes of the formula (II)

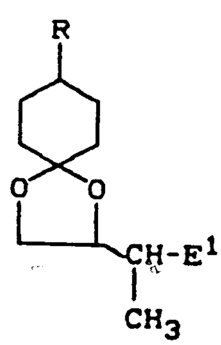

(II)

in which

R represents alkyl, or represents in each case optionally substituted cycloalkylalkyl, aralkyl, cycloalkyl or aryl and

$E^1$ represents an electron-withdrawing leaving group.

characterized in that cyclohexanone derivatives of the formula (V)

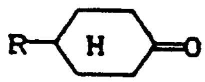

(V)

in which

R has the abovementioned meaning,

either

(a) are cyclized with alcohols of the formula (VIa)

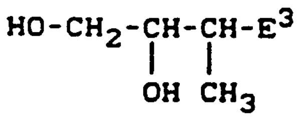

(VIa)

in which

$E^3$ represents halogen or hydroxyl,

if appropriate in the presence of a diluent and if appropriate in the presence of an acid catalyst, at temperatures between 40 °C and 150 °C,

or

(b) are reacted with oxiranyl compounds of the formula (VIb)

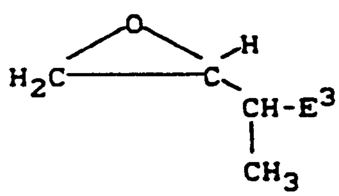

(VIb)

in which

$E^3$ represents halogen or hydroxyl,

if appropriate in the presence of a diluent and if appropriate in the presence of a catalyst, at temperatures between -20 °C and +40 °C, and if appropriate in the cases where $E^3$ in formula (VIa) or (VIb) represents a hydroxyl group, the hydroxymethyldioxolanes thus obtainable, of the formula (VII)

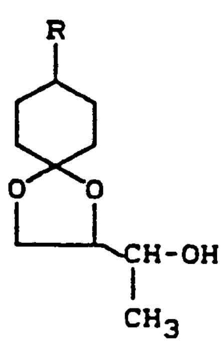

(VII)

in which

R has the abovementioned meaning,

are reacted in a 2nd stage with optionally substituted alkyl- or arylsulphonyl halides of the formula (VIII)

$Z\text{-}SO_2\text{-}Hal$ (VIII)

in which

Hal represents halogen, and

Z represents alkyl, in each case optionally substituted by halogen or represents aryl which is optionally substituted by alkyl having 1 to 4 carbon atoms,

if appropriate in the presence of a diluent and if appropriate in the presence of an acid-binding agent, at temperatures between -20 °C and +100 °C.

## Revendications

**1.** Dioxolannyléthylamines substituées de formule générale (I),

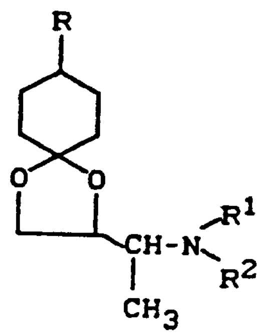

(I)

dans laquelle

R représente un groupe alkyle ou un groupe cycloalkylalkyle, aralkyle, cycloalkyle ou aryle dont chacun est éventuellement substitué,

$R^1$ et $R^2$ représentent chacun indépendamment l'un de l'autre l'hydrogène, un groupe alkyle, alcényle, alcynyle, alkoxyalkyle, dialkoxyalkyle, hydroxyalkyle, hydroxyalkoxyalkyle, alkoxycarbonylalkyle, dioxolannylalkyle, dioxannylalkyle, oxolannylalkyle ou un groupe cycloalkylalkyle, cycloalkyle, aralkyle, aralcényle ou aryle portant chacun le cas échéant un substituant, ou bien

$R^1$ et $R^2$ forment conjointement avec l'atome d'azote auquel ils sont liés un hétérocycle saturé éventuellement substitué qui peut contenir le cas échéant d'autres hétéro-atomes,

et leurs sels d'addition d'acides.

**2.** Dioxolannyléthylamines substituées suivant la revendication 1, dans la formule (I) desquelles

R est un groupe alkyle à chaîne droite ou ramifiée ayant 1 à 12 atomes de carbone, un groupe cycloalkylalkyle ou cycloalkyle ayant chacun 3 à 7 atomes de carbone dans la partie cycloalkyle et le cas échéant 1 à 6 atomes de carbone dans la partie alkyle à chaîne droite ou ramifiée, portant chacun le cas échéant un ou plusieurs substituants, identiques ou différents, halogéno et/ou alkyle à chaîne droite ou ramifiée en $C_1$ à $C_8$ ou un groupe aralkyle ou aryle ayant chacun 6 à 10 atomes de carbone dans la partie aryle et le cas échéant 1 à 6 atomes de carbone dans la partie alkyle à chaîne droite ou ramifiée, portant chacun le cas échéant un ou plusieurs substituants, identiques ou différents, halogéno et alkyle à chaîne droite ou ramifiée ayant 1 à 8 atomes de carbone et

$R^1$ et $R^2$ représentent chacun indépendamment l'un de l'autre l'hydrogène, un groupe alkyle ayant 1 à 12 atomes de carbone, un groupe alcényle ayant 3 à 8 atomes de carbone, un groupe alcynyle ayant 3 à 8 atomes de carbone, un groupe hydroxyalkyle ayant 2 à 6 atomes de carbone, un groupe alkoxyalkyle ou dialkoxyalkyle ayant chacun 1 à 6

atomes de carbone, chacun à chaîne droite ou ramifiée, ou un groupe hydroxyalkoxyalkyle ayant 2 à 6 atomes de carbone dans les parties alkyle individuelles, un groupe alkoxycarbonylalkyle ayant 1 à 6 atomes de carbone par partie alkoxy ou alkyle, un groupe dioxolannylalkyle, dioxannylalkyle ou oxolannylalkyle chacun à chaîne droite ou ramifiée et ayant chacun 1 à 4 atomes de carbone dans la partie alkyle ou un groupe cycloalkyle ou cycloalkylalkyle ayant chacun 3 à 7 atomes de carbone dans la partie cycloalkyle et le cas échéant 1 à 4 atomes de carbone dans la partie alkyle à chaîne droite ou ramifiée, portant chacun le cas échéant dans la partie cycloalkyle un ou plusieurs substituants identiques ou différents, en considérant dans chaque cas comme substituants : un halogène, un groupe alkyle, alkoxy, halogénalkyle ou halogénalkoxy chacun à chaîne droite ou ramifiée et ayant chacun 1 à 4 atomes de carbone et le cas échéant 1 à 9 atomes d'halogènes identiques ou différents ; en outre un groupe arylalkyle, arylalcényle ou aryle ayant chacun 6 à 10 atomes de carbone dans la partie aryle et le cas échéant jusqu'à 6 atomes de carbone dans la partie alkyle ou alcényle à chaîne droite ou ramifiée, portant chacun le cas échéant un ou plusieurs substituants identiques ou différents dans la partie aryle, en considérant dans chaque cas comme substituants de la partie aryle : un halogène, un groupe cyano, nitro, un groupe alkyle, alkoxy, alkylthio, halogénalkyle, halogénalkoxy, halogénalkylthio, alkoxycarbonyle ou alkoximinoalkyle chacun à chaîne droite ou ramifiée et ayant chacun 1 à 4 atomes de carbone dans les parties alkyle individuelles et le cas échéant 1 à 9 atomes d'halogènes identiques ou différents, ou bien

$R^1$ et $R^2$ forment conjointement avec l'atome d'azote auquel ils sont liés un hétérocycle pentagonal à heptagonal saturé portant le cas échéant un ou plusieurs substituants identiques ou différents, qui peut contenir éventuellement un autre hétéroatome, et on considère alors comme substituants : un groupe alkyle ou hydroxyalkyle, chacun à chaîne droite ou ramifiée, avec dans chaque cas 1 à 4 atomes de carbone.

**3.** Dioxolannyléthylamines substituées suivant la revendication 1, dans lesquelles

R est un groupe alkyle à chaîne droite ou ramifiée ayant 1 à 8 atomes de carbone, un groupe cyclohexyle portant le cas échéant 1 à 3 substituants, identiques ou différents, chlore, méthyle, éthyle, n- ou isopropyle, n-, iso-, sec.- ou tertio-butyle, un groupe cyclohexylalkyle ayant 1 à 4 atomes de carbone dans la partie alkyle à chaîne droite ou ramifiée, portant éventuellement 1 à 3 substituants, identiques ou différents, chloro, méthyle, éthyle, n-propyle, isopropyle, n-butyle, isobutyle, sec.-butyle ou tertio-butyle, un groupe phényle portant le cas échéant 1 à 3 substituants, identiques ou différents, fluoro, chloro, bromo, méthyle, éthyle, n-propyle, iso-propyle, n-butyle, isobutyle, sec.-butyle ou tertio-butyle ou un groupe phénylalkyle ayant 1 à 4 atomes de carbone dans la partie alkyle à chaîne droite ou ramifiée, portant le cas échéant 1 à 3 substituants, identiques ou différents, fluoro, chloro, bromo, méthyle, éthyle, n-propyle, iso-propyle, n-butyle, iso-butyle, sec.-butyle ou tertio-butyle et

$R^1$ et $R^2$ représentent chacun indépendamment l'un de l'autre l'hydrogène, un groupe méthyle, éthyle, n-propyle, isopropyle, n-butyle, isobutyle, sec.-butyle, tertiobutyle, n-pentyle, isopentyle, n-hexyle, iso-hexyle, n-heptyle, isoheptyle, n-octyle, iso-octyle, allyle, n-butényle, iso-butényle, n-pentényle, iso-pentényle, propargyle, n-butynyle, iso-butynyle, hydroxyéthyle, hydroxypropyle, méthoxyéthyle, éthoxyéthyle, propoxyéthyle, butoxyéthyle, méthoxypropyle, éthoxypropyle, propoxypropyle, butoxypropyle, hydroxyéthoxyéthyle, diméthoxyéthyle, diméthoxypropyle, diéthoxyéthyle, méthoxycarbonylméthyle, méthoxycarbonyléthyle, méthoxycarbonylpropyle, éthoxycarbonylméthyle, éthoxycarbonyléthyle, éthoxycarbonylpropyle, propoxycarbonylméthyle, propoxycarbonyléthyle, propoxycarbonylpropyle, dioxolannylméthyle, dioxolannyléthyle, dioxannylméthyle, dioxannyléthyle, oxolannylméthyle, oxolannyléthyle, un groupe cyclopropyle, cyclopropylméthyle, cyclopropyléthyle, cyclopropylpropyle, cyclopentyle,cyclopentylméthyle, cyclohexyle ou cyclohexylméthyle portant chacun le cas échéant un à cinq substituants, identiques ou différents, fluoro, chloro, bromo, méthyle, éthyle, n-propyle, isopropyle, n-butyle, isobutyle, sec.-butyle, tertio-butyle, trifluorométhyle ou trifluorométhoxy, ou un groupe phényle, benzyle ou phényléthyle portant chacun le cas échéant un à trois substituants identiques ou différents, en considérant dans chaque cas comme substituants : un radical fluoro, chloro, bromo, cyano, nitro, méthyle, éthyle, n-propyle,

isopropyle, n-butyle, isobutyle, sec.-butyle, tertio-butyle, méthoxy, éthoxy, méthylthio, trifluorométhyle, trifluorométhoxy, trifluorométhylthio, méthoxycarbonyle, éthoxycarbonyle ou méthoximinométhyle ou bien

$R^1$ et $R^2$ forment conjointement avec l'atome d'azote auquel ils sont liés un hétérocycle de formule

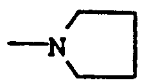 ; 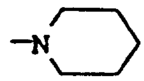 ; 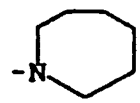 ; 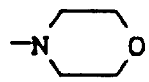

ou 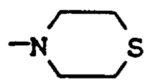

portant le cas échéant un à trois substituants identiques ou différents, en considérant comme substituants dans chaque cas un radical méthyle, éthyle ou hydroxyméthyle.

**4.** Dioxolannyléthylamines substituées suivant la revendication 1, dans la formule (I) desquelles

R est un groupe cyclohexyle, phényle ou l'un des restes

$-CH_2-R^3$, 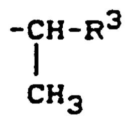 ou 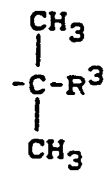 ,

dans lesquels

$R^3$ désigne dans chaque cas l'hydrogène, un groupe méthyle, éthyle, n-propyle, isopropyle, n-butyle, isobutyle, sec.-butyle, tertio-butyle, néopentyle, cyclohexyle ou phényle,

$R^1$ et $R^2$ représentent chacun indépendamment l'un de l'autre l'hydrogène, un groupe méthyle, éthyle, n-propyle, isopropyle, n-butyle, isobutyle, sec.-butyle, tertiobutyle, n-pentyle, iso-pentyle, n-hexyle, iso-hexyle, allyle, n-butényle, isobutényle, n-pentényle, isopentényle, propargyle, n-butynyle, isobutynyle, hydroxyéthyle, hydroxypropyle, méthoxyéthyle, méthoxypropyle, éthoxyéthyle, éthoxypropyle, hydroxyéthoxyéthyle, diméthoxyéthyle, diéthoxyéthyle, méthoxycarbonylméthyle, méthoxycarbonyléthyle, méthoxycarbonylpropyle, éthoxycarbonylméthyle, éthoxycarbonyléthyle,éthoxycarbonylpropyle, propoxycarbonylméthyle, propoxycarbonyléthyle, propoxycarbonylpropyle, dioxolannylméthyle, dioxolannyléthyle, dioxannylméthyle, oxolannylméthyle, oxolannyléthyle, cyclopropylméthyle, dichlorocyclopropylméthyle, diméthylcyclopropylméthyle, dichlorodiméthylcyclopropylméthyle, cyclopentyle, cyclohexyle ou cyclohexylméthyle ou bien

$R^1$ et $R^2$ forment conjointement avec l'atome d'azote auquel ils sont liés un hétérocycle de formule

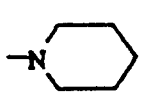 ; 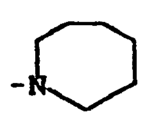 ; 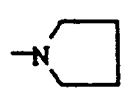 ou 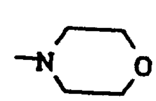

portant le cas échéant un à trois substituants identiques ou différents, en considérant dans chaque cas comme substituants les groupes méthyle, éthyle ou hydroxyméthyle.

**5.** Procédé de production de dioxolannyléthylamines substituées de formule générale (I),

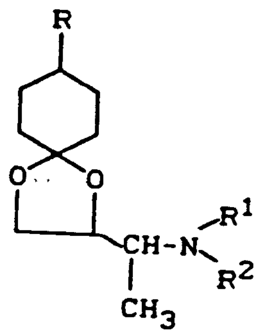

(I)

dans laquelle

R représente un groupe alkyle ou un groupe cycloalkylalkyle, aralkyle, cycloalkyle ou aryle dont chacun est éventuellement substitué,

$R^1$ et $R^2$ représentent chacun indépendamment l'un de l'autre l'hydrogène, un groupe alkyle, alcényle, alcynyle, alkoxyalkyle, dialkoxyalkyle, hydroxyalkyle, hydroxyalkoxyalkyle, alkoxycarbonylalkyle, dioxolannylalkyle, dioxannylalkyle, oxolannylalkyle ou un groupe cycloalkylalkyle, cycloalkyle, aralkyle, aralcényle ou aryle portant chacun le cas échéant un substituant, ou bien

$R^1$ et $R^2$ forment conjointement avec l'atome d'azote auquel ils sont liés un hétérocycle saturé éventuellement substitué qui peut contenir le cas échéant d'autres hétéro-atomes,

et de leurs sels d'addition d'acides, caractérisé en ce que

(a) on fait réagir des dioxolannes substitués de formule (II),

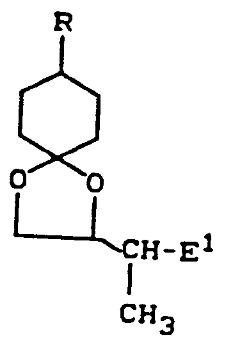

(II)

dans laquelle

R a la définition indiquée ci-dessus et

$E^1$ est un groupe partant attirant les électrons, avec des amines de formule (III),

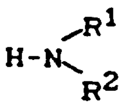

(III)

dans laquelle

$R^1$ et $R^2$ ont la définition indiquée ci-dessus,

le cas échéant en présence d'un diluant et en la présence éventuelle d'un accepteur d'acide, ou bien

(b) on fait réagir les dioxolannyléthylamines substituées conformes à l'invention, obtenues par le procédé (a), de formule (Ia),

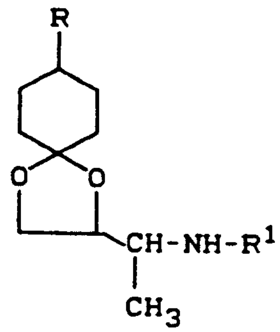

(Ia)

dans laquelle

R et $R^1$ ont la définition indiquée ci-dessus,

avec des agents d'alkylation de formule (IV),

$R^{2-1}$-$E^2$ (IV)

dans laquelle

$R^{2-1}$ représente un groupe alkyle, alcényle, alcynyle, alkoxyalkyle, dialkoxyalkyle, hydroxyalkyle, hydroxyalkoxyalkyle, alkoxycarbonylalkyle, dioxolannylalkyle, dioxannylalkyle, oxolannylalkyle ou un groupe cycloalkylalkyle, cycloalkyle, aralkyle ou aralcényle, chacun étant éventuellement substitué, et

$E^2$ est un groupe partant attirant les électrons,

le cas échéant en présence d'un diluant et en la présence éventuelle d'un accepteur d'acide et on additionne éventuellement un acide ou bien on fait suivre une opération de séparation physique.

**6.** Compositions destinées à combattre des parasites, caractérisées par une teneur en au moins une dioxolannyléthylamine substituée de formule (I) suivant les revendication 1 ou 5.

**7.** Utilisation de dioxolannyléthylamines substituées de formule (I) suivant les revendications 1 ou 5 pour combattre des parasites.

**8.** Procédé pour combattre des parasites, caractérisé en ce qu'on fait agir au moins une dioxolannyléthylamine substituée de formule (I) suivant les revendications 1 ou 5 sur les parasites et/ou sur leur habitat.

**9.** Procédé de préparation de compositions destinées à la lutte contre des parasites, caractérisé en ce qu'on mélange au moins une dioxolannyléthylamine substituée de formule (I) suivant les revendications 1 ou 5 avec des diluants et/ou des agents tensio-actifs.

**10.** Dioxolannes substitués de formule (II),

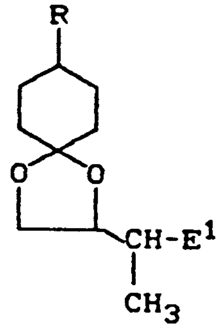

(II)

dans laquelle

R est un groupe alkyle ou un groupe cycloalkylalkyle, aralkyle, cycloalkyle ou aryle chacun éventuellement substitué et

$E^1$ est un groupe partant attirant les électrons.

**11.** Procédé de production de dioxolannes substitués de formule (II),

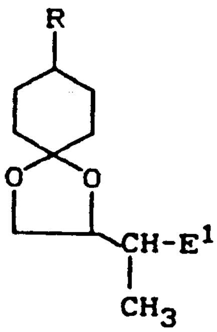

(II)

dans laquelle

R est un groupe alkyle ou un groupe cycloalkylalkyle, aralkyle, cycloalkyle ou aryle chacun éventuellement substitué et

$E^1$ est un groupe partant attirant les électrons, caractérisé en ce que des dérivés de cyclohexanones de formule (V),

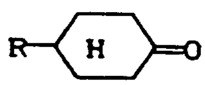

(V)

dans laquelle

R a la définition indiquée ci-dessus,

(a) sont cyclisés avec des alcools de formule (VIa),

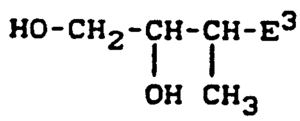

(VIa)

dans laquelle

$E^3$ est un halogène ou un groupe hydroxy,

le cas échéant en présence d'un diluant et en la présence éventuelle d'un catalyseur acide à des températures comprises entre 40 °C et 150 °C

ou bien

(b) sont amenés à réagir avec des composés d'oxirannyle de formule (VIb),

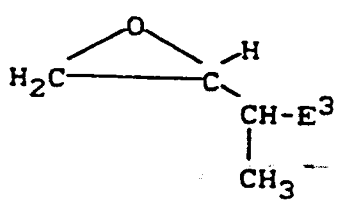

(VIb)

dans laquelle

$E^3$ est un halogène ou un groupe hydroxy,le cas échéant en présence d'un diluant et en la présence éventuelle d'un catalyseur, à des températures comprises entre -20 °C et +40 °C et dans les cas éventuels où $E^3$ dans la formule (VIa) ou (VIb) représente un groupe hydroxy, on fait réagir dans une seconde étape, à des températures comprises entre -20 °C et +100 °C, le cas échéant en présence d'un diluant et en la présence éventuelle d'un accepteur d'acide, les hydroxyméthyldioxolannes ainsi obtenus de formule (VII),

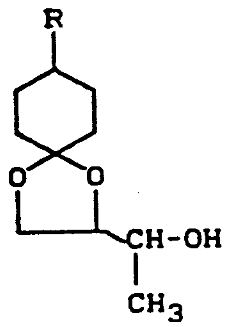

(VII)

dans laquelle

R a la définition indiquée ci-dessus, avec des halogénures d'alkyl- ou d'arylsulfonyle éventuellement substitués, de formule (VIII),

$Z\text{-}SO_2\text{-}Hal$ (VIII)

dans laquelle

Hal représente un halogène et

Z est un groupe alkyle éventuellement substitué dans chaque cas par un halogène ou un groupe aryle éventuellement substitué par un groupe alkyle ayant 1 à 4 atomes de carbone.